# EUROPEAN PATENT APPLICATION

(11) **EP 3 521 892 A1**
(43) Date of publication of application: **07.08.2019**
(21) Application number: 17855838.3
(22) Date of filing: 19.09.2017
(51) Int. Cl.: G02B 21/26, C12M 1/34, G01N 21/17, G02B 21/06, G02B 21/36

(54) **OBSERVATION DEVICE**

(30) Priority: 29.09.2016 JP 2016191204
(71) Applicant: Olympus Corporation, Hachioji-shi Tokyo 192-8507 (JP)
(72) Inventor: TAKAHASHI, Shintaro, Hachioji-shi Tokyo 192-8507 (JP); TAKIMOTO, Shinichi, Hachioji-shi Tokyo 192-8507 (JP); TANIKAWA, Yohei, Hachioji-shi Tokyo 192-8507 (JP); MATSUSHITA, Akira, Hachioji-shi Tokyo 192-8507 (JP); MIZUNAKA, Masaru, Hachioji-shi Tokyo 192-8507 (JP); MOCHIZUKI, Tsuyoshi, Hachioji-shi Tokyo 192-8507 (JP)
(74) Representative: Winter, Brandl, Fürniss, Hübner, Röss, Kaiser, Polte - Partnerschaft mbB
(86) International application number: PCT/JP2017/033764
(87) International publication number: WO 2018/061883

(57) **Abstract**

For the purpose of suppressing interference with work even when being used in a limited space as in a clean bench, thus improving work efficiency, an observation device (1) of the present invention includes: a housing (5) having, in a top surface, a glass window (5b) on which a vessel (3) accommodating samples (X) is placed and through which illumination light can be transmitted; and an image acquisition unit (9) that is accommodated in the housing (5) and that captures transmitted light obtained after illumination light is transmitted through the samples (X) from above, is transmitted through the glass window (5b), and enters the housing (5).

## Description

### {Technical Field}

The present invention relates to an observation device.

### {Background Art}

In the related art, there is a known observation device for sample observation, to be mounted in a clean bench (for example, see PTLs 1 and 2).

### {Citation List}

### {Patent Literature}

{PTL 1} Japanese Unexamined Patent Application, Publication No. 2001-25387
{PTL 2} Japanese Unexamined Patent Application, Publication No. 2009-106305

### {Summary of Invention}

### {Technical Problem}

However, because the observation device described in PTL 1, 2 is a general inverted microscope supported by a support stand, there is a disadvantage in that the support stand, an image-acquisition optical system, etc. interfere with work when the microscope is used in a limited workspace in a clean bench.

The present invention has been made in view of the above-described circumstances, and an object thereof is to provide an observation device that does not interfere with work even when the observation device is used in a limited space as in a clean bench, thus making it possible to improve work efficiency.

### {Solution to Problem}

In order to achieve the above-described object, the present invention provides the following solutions.

According to one aspect, the present invention provides an observation device including: a housing having, in a top surface, a transmissive window on which a vessel accommodating samples can be placed and through which illumination light can be transmitted; and an image acquisition unit that is accommodated in the housing and that captures transmitted light obtained after the illumination light is transmitted through the samples from above, is transmitted through the transmissive window, and enters the housing.

According to this aspect, when illumination light is radiated onto the samples in the vessel, which is placed on the transmissive window in the top surface of the housing, from above, transmitted light transmitted through the samples downward from above is also transmitted through the transmissive window, thus entering the housing, and is captured by the image acquisition unit in the housing. The image acquisition unit is accommodated in the housing, on which the vessel is placed, thereby suppressing interference of the image acquisition unit with work even when the observation device is used in a limited space as in a clean bench, thus making it possible to improve work efficiency.

The above-described aspect may further include a light source unit that is accommodated in the housing and that emits the illumination light upward from below the samples, wherein the image acquisition unit may capture the transmitted light transmitted through the samples and the transmissive window after the illumination light emitted by the light source unit is reflected above the samples.

With this configuration, the light source unit is accommodated in the housing, on which the vessel is placed, thereby making it possible to suppress interference of the light source unit with work. In this case, the light source unit and the image acquisition unit are both disposed below the samples, thereby making it possible to reduce the height of the device and to make the housing thinner, compared with a case in which the light source unit and the image acquisition unit are separately disposed above and below the samples with the samples sandwiched therebetween. Accordingly, even when the observation device is used in a limited space as in a clean bench, it is possible to improve work efficiency.

In the above-described aspect, the image acquisition unit may include an objective lens that focuses the transmitted light, and the light source unit may emit the illumination light from a radially outer side of the objective lens toward an upper side of the samples.

With this configuration, illumination light emitted by the light source unit, which is disposed on the radially outer side of the objective lens disposed below the samples, toward the upper side of the samples is reflected above the samples and is incident on the samples from obliquely above with respect to the optical axis of the objective lens, and transmitted light transmitted through the samples is captured by the image acquisition unit. By appropriately setting the incidence angle to the samples, it is possible to form light and dark areas in an image of the samples and to acquire an image that is easy to see even in the case of a transparent subject, such as cells.

In the above-described aspect, the light source unit may be able to independently emit the illumination light from a different position in a radial direction of the objective lens.

With this configuration, the radial position of illumination light emitted by the light source unit is made different, thereby making it possible to change the incidence angle, to the samples, of reflected light reflected at the same reflective surface disposed above the samples. Specifically, reflected light of illumination light emitted from a position near the objective lens in the radial direction is incident on the samples at a small angle with respect to the optical axis, whereas reflected light of illumination light emitted from a position far from the objective lens in the radial direction is incident on the samples at a large angle with respect to the optical axis.

Accordingly, in a case in which the incidence angle is smaller than the capturing angle of the objective lens, bright-field illumination in which there is less unevenness in illumination can be provided, in a case in which the incidence angle is larger than the capturing angle of the objective lens, dark-field illumination in which the microstructure is emphasized can be provided, and, in a case in which the incidence angle is equivalent to the capturing angle of the objective lens, focal illumination in which the samples look three-dimensional can be provided.

In the above-described aspect, the light source unit may be able to simultaneously emit the illumination light from different positions in a circumferential direction of the objective lens.

With this configuration, illumination light is simultaneously radiated from a plurality of positions in the circumferential direction of the objective lens, thus making it possible to reduce unevenness in illumination.

In the above-described aspect, the light source unit may include a plurality of light sources that are arranged around the objective lens and that can be independently turned on.

With this configuration, by turning on any of the plurality of light sources, the circumferential position of illumination light can be determined. Then, by changing the circumferential position of the light source to be turned on, it is possible to capture an image of the samples illuminated from a different position. In particular, in the above-described focal illumination, it is possible to capture images in which there are different shadows.

In the above-described aspect, the light source unit may include: a light source that is disposed below the samples; and a light shielding member having an opening that allows, of the illumination light from the light source, only the illumination light at a particular radial position to pass therethrough.

With this configuration, illumination light from the light source is shielded by the light shielding member, and only illumination light that passes through the opening is reflected above the samples and is incident on the samples. Therefore, by adjusting the position of the opening of the light shielding member, without changing the position of the light source to be turned on, it is possible to change the direction or the angle of the reflected light incident on the samples.

In the above-described aspect, the image acquisition unit may include: a camera unit that receives the illumination light focused by the objective lens and that acquires an image of the samples; and an optical filter that allows only a wavelength of the illumination light to pass therethrough, the objective lens may be disposed so as to be opposed to the samples with the transmissive window sandwiched therebetween, and the optical filter may be disposed at one of a position between the transmissive window and the objective lens, a position inside the objective lens, and a position between the objective lens and the camera unit.

With this configuration, because light, other than illumination light, that is about to enter the camera unit is cut off by the optical filter, it is possible to reduce deterioration of image quality caused by the influence of the light other than illumination light.

In the above-described aspect, the light source unit may include a diffusion plate that diffuses the illumination light.

With this configuration, illumination light evenly diffused by the diffusion plate can be radiated onto the samples.

In the above-described aspect, the illumination light may be reflected by an inner surface of a top plate of the vessel.

With this configuration, merely by disposing the vessel, which has the top plate and which accommodates the samples therein, above the light source unit and the image acquisition unit, illumination light emitted by the light source unit can be reflected at the inner surface of the top plate of the vessel and can be radiated onto the samples in the vessel.

In the above-described aspect, the illumination light may be reflected by a reflecting member disposed above the samples.

With this configuration, in a case of observing samples accommodated in a cell culture bag or in a vessel in which observation may be performed in a state where the top plate is removed, as in a petri dish, the reflecting member is disposed above the samples, so that illumination light emitted by the light source unit can be reflected at the reflecting member and can be radiated onto the samples in the vessel.

In the above-described aspect, the samples may be immersed in a solution, and the illumination light may be reflected by an upper liquid surface of the solution.

With this configuration, in a case of observing samples accommodated in a vessel that does not have a top plate or in a vessel for which a reflecting member cannot be disposed, illumination light emitted by the light source unit can be reflected at the liquid surface of the solution and can be radiated onto the samples in the vessel.

In the above-described aspect, the image acquisition unit may capture the transmitted light transmitted through the samples and the transmissive window after the illumination light emitted by a light source disposed outside the housing is radiated onto the samples from above.

With this configuration, the light source is not accommodated in the housing, so that the housing can be reduced in thickness and size.

In the above-described aspect, the image acquisition unit may include: an objective lens that focuses the transmitted light; a camera unit that receives the illumination light focused by the objective lens and that acquires an image of the samples; and an optical filter that allows only a wavelength of the illumination light to pass therethrough, the objective lens may be disposed so as to be opposed to the samples with the transmissive window sandwiched therebetween, and the optical filter may be disposed at one of a position between the transmissive window and the objective lens, a position inside the objective lens, and a position between the objective lens and the camera unit.

With this configuration, because light, other than illumination light, that is about to enter the camera unit is cut off by the optical filter, it is possible to reduce deterioration of image quality caused by the influence of the light other than illumination light.

The above-described aspect may further include a display unit that displays an image of the samples acquired by the image acquisition unit.

With this configuration, the user can observe the samples by viewing an image displayed on the display unit.

In the above-described aspect, the display unit may be disposed in one surface of the housing.

With this configuration, a space for disposing the display unit separately from the housing can be saved.

In the above-described aspect, the display unit may be a projector that projects the image.

With this configuration, a space for disposing the display unit separately from the housing can be saved, and the projector projects an image at a position that the user can easily see in a posture adopted while working, thus making it possible to improve work efficiency.

The above-described aspect may further include: a focus adjustment unit that adjusts a focus state of the image acquisition unit with respect to the samples; and a control unit that controls the focus adjustment unit so as to match a focal point of the image acquisition unit with the samples.

With this configuration, the focal point of the objective lens can be automatically adjusted via the focus adjustment unit by the control unit. By eliminating work performed manually, it is more effective when the observation device is used in a limited space as in a clean bench.

In the above-described aspect, at least part of the control unit may be contained in the housing.

With this configuration, the control unit is contained in the housing, so that a space outside the housing can be secured.

The above-described aspect may further include a manipulation unit that enables a user to input an instruction to be sent to the control unit.

With this configuration, the user merely inputs an instruction to the manipulation unit, thereby making it possible to carry out a desired manipulation.

In the above-described aspect, the manipulation unit may be a foot switch.

With this configuration, the user can input an instruction to the manipulation unit while doing other work with his or her hands, thus making it possible to improve work efficiency.

In the above-described aspect, the manipulation unit may be disposed on one surface of the housing.

With this configuration, a space for disposing the manipulation unit separately from the housing can be saved.

In the above-described aspect, the top surface of the housing may be disposed so as to be substantially horizontal.

With this configuration, the top surface of the housing can be used as a workbench during non-observation, thus making it possible to effectively secure the workspace.

The above-described aspect may further include an image aggregation unit that puts an image of the samples acquired by the image acquisition unit together with an image acquired by another observation device and that stores the images in an external recording medium.

With this configuration, by means of the image aggregation unit, an image of the samples acquired by the image acquisition unit and an image acquired by the other observation device can be easily managed together in the external recording medium. Therefore, when the images are read from the external recording medium, the samples to be observed by this observation device can be observed together with the samples in the other observation device while being compared with them.

### {Advantageous Effects of Invention}

According to a microscope of the present invention, an advantageous effect is afforded in that, even when the microscope is used in a limited space as in a clean bench, the microscope does not interfere with work, thus making it possible to improve work efficiency.

### {Brief Description of Drawings}

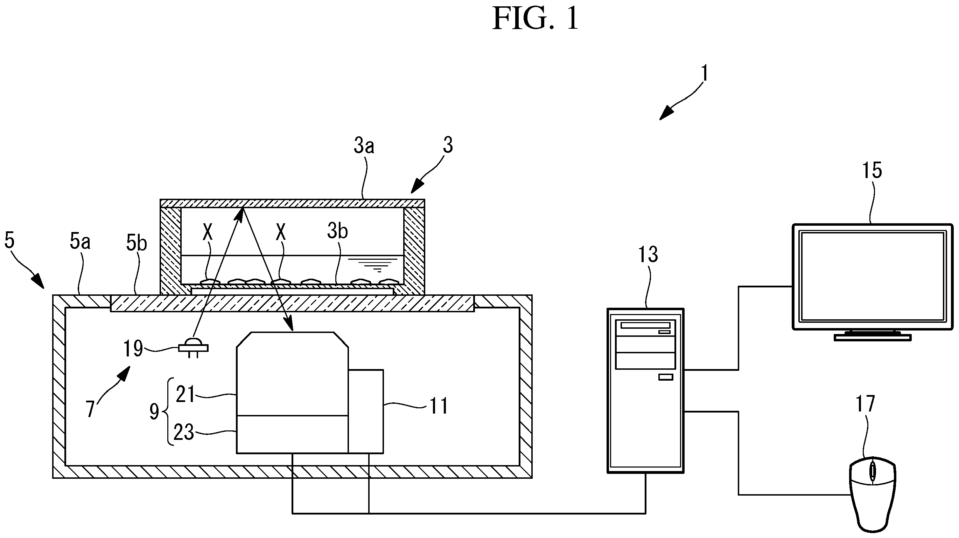

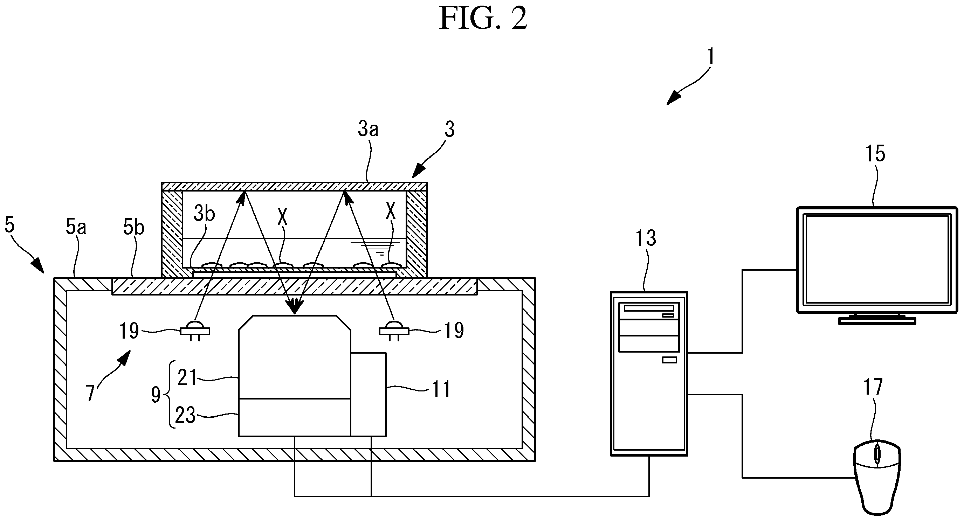

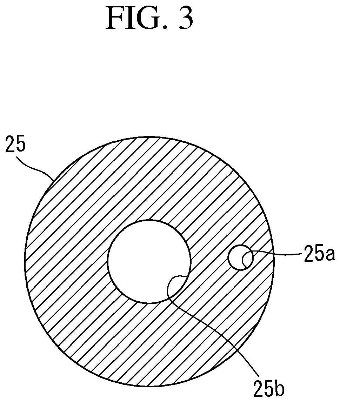

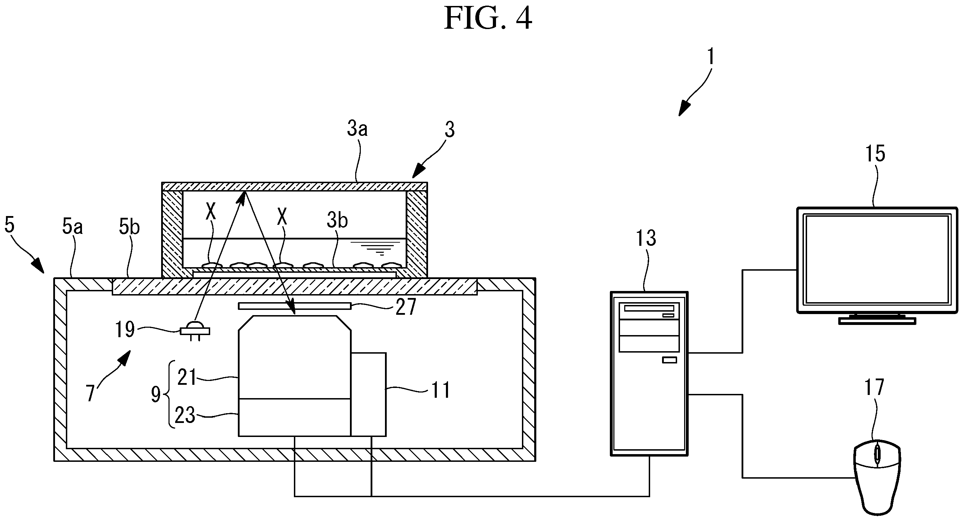

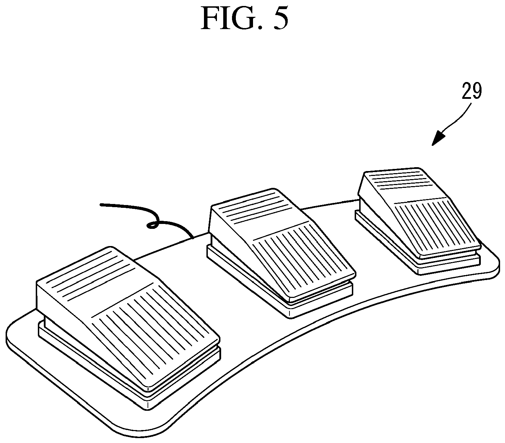

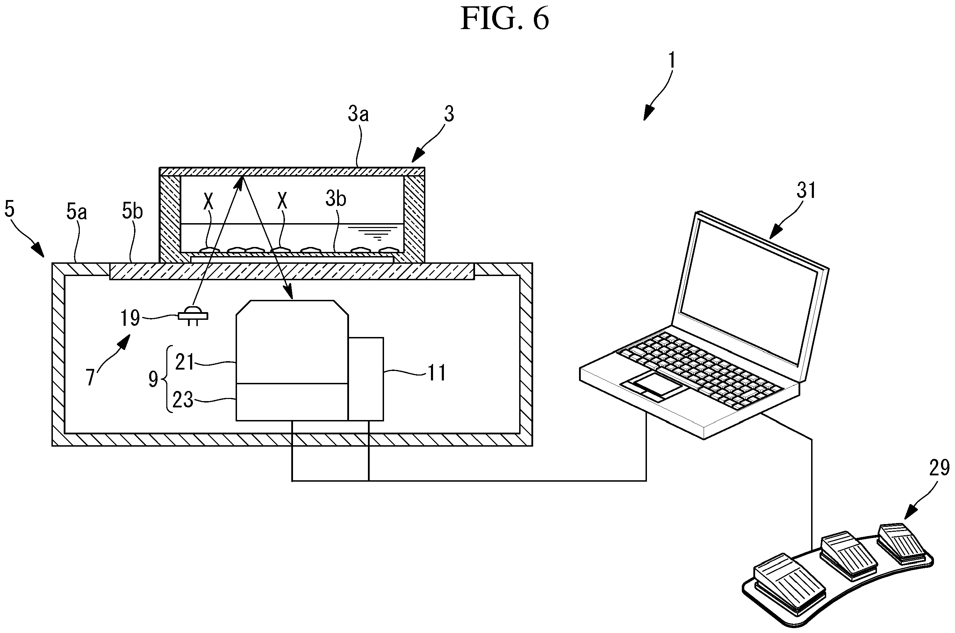

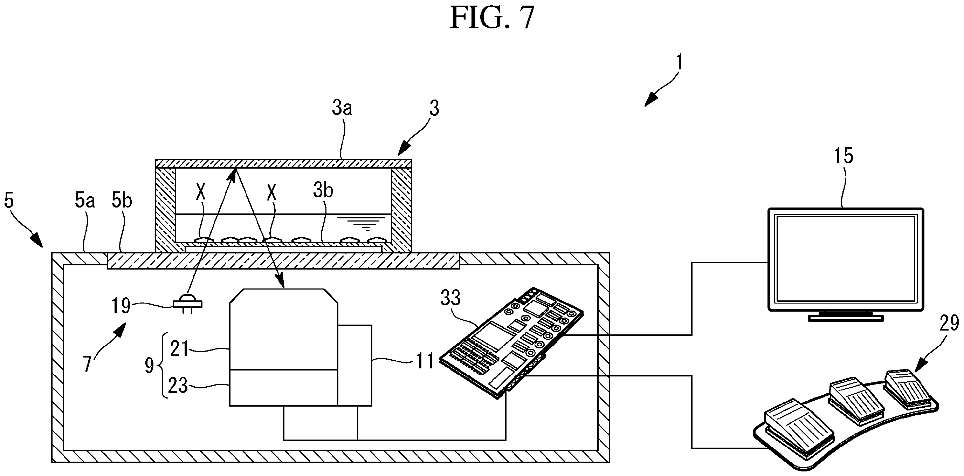

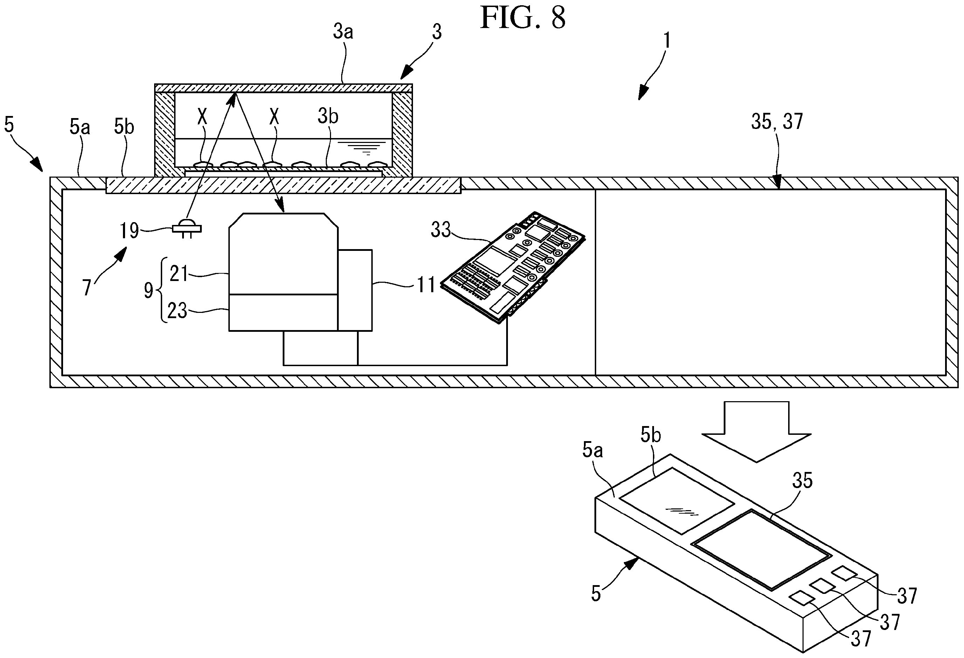

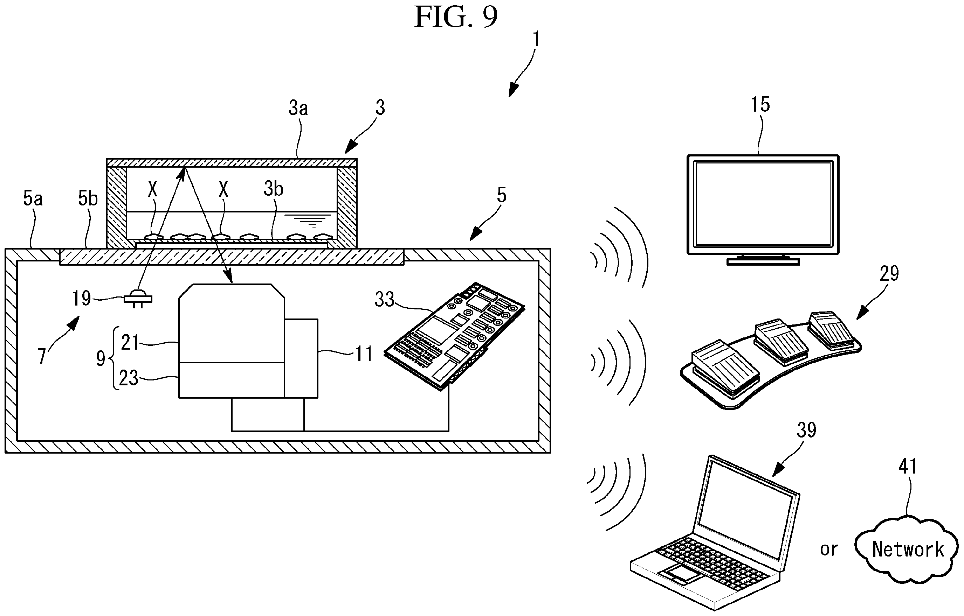

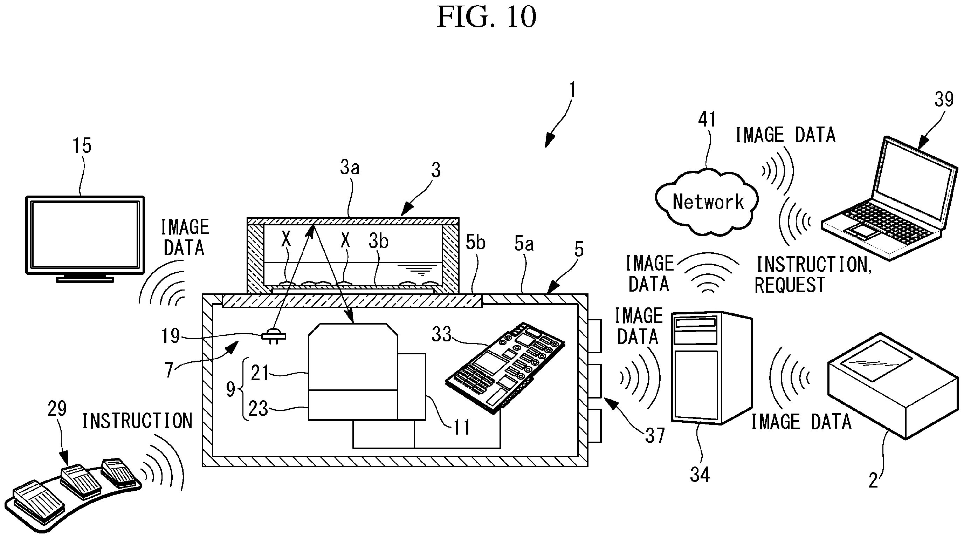

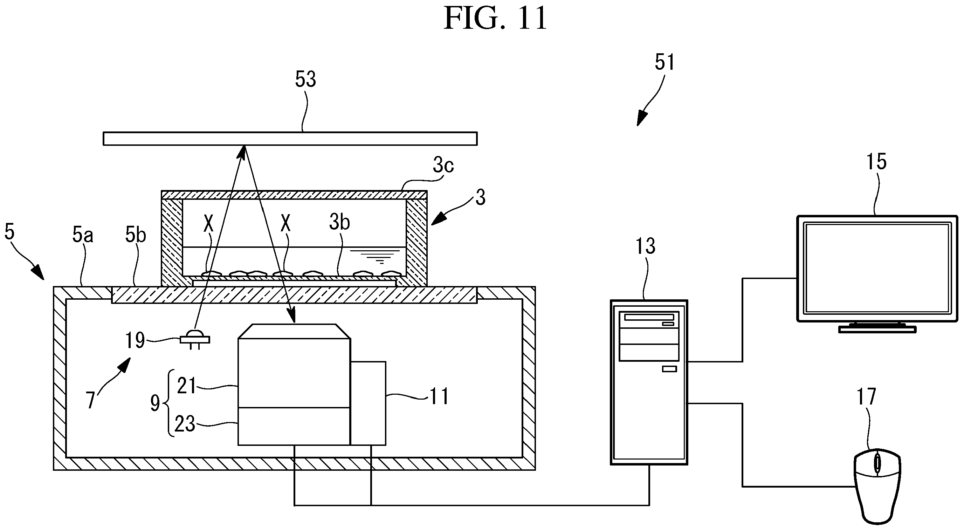

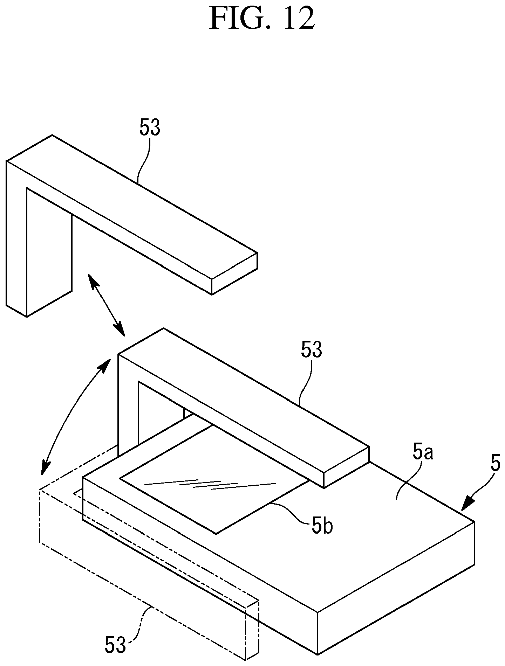

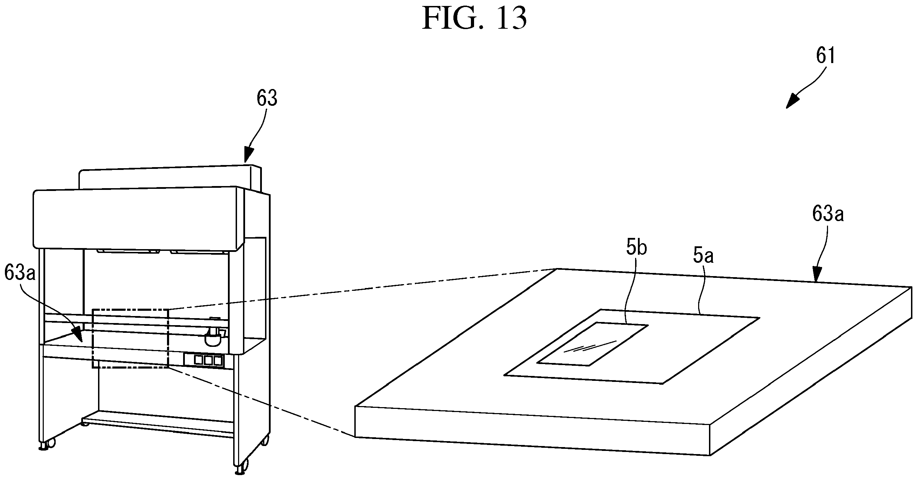

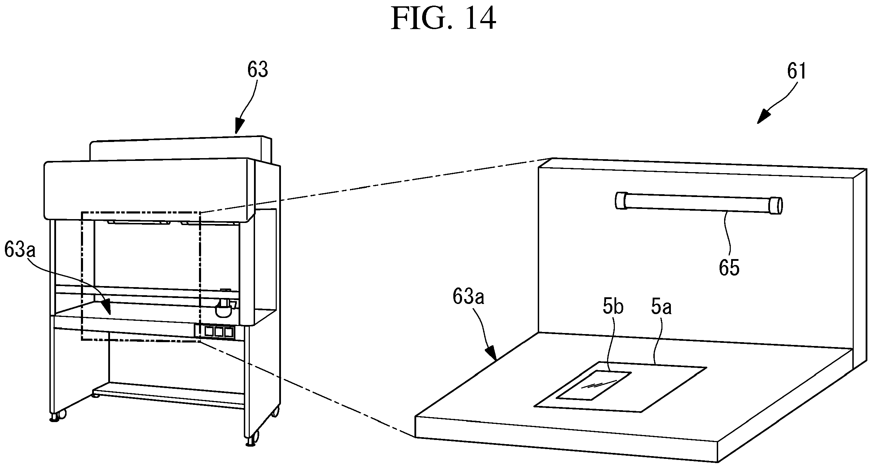

{Fig. 1} Fig. 1 is a view showing, in outline, the configuration of an observation device according to a first embodiment of the present invention.
{Fig. 2} Fig. 2 is a view showing, in outline, the configuration of an observation device according to a first modification of the first embodiment of the present invention.
{Fig. 3} Fig. 3 is a plan view showing an example light shielding member having a single circular opening.
{Fig. 4} Fig. 4 is a view showing, in outline, the configuration of an observation device according to a second modification of the first embodiment of the present invention.
{Fig. 5} Fig. 5 is a view showing an example manipulation unit, such as a foot switch, that is manipulated by a user with his or her foot.
{Fig. 6} Fig. 6 is a view showing, in outline, the configuration of an observation device according to a fourth modification of the first embodiment of the present invention.
{Fig. 7} Fig. 7 is a view showing, in outline, the configuration of an observation device according to a fifth modification of the first embodiment of the present invention.
{Fig. 8} Fig. 8 is a view showing, in outline, the configuration of an observation device according to a sixth modification of the first embodiment of the present invention.
{Fig. 9} Fig. 9 is a view showing, in outline, the configuration of an observation device according to a seventh modification of the first embodiment of the present invention.
{Fig. 10} Fig. 10 is a schematic view showing an observation device according to an eighth modification of the first embodiment of the present invention.
{Fig. 11} Fig. 11 is a view showing, in outline, the configuration of an observation device according to a second embodiment of the present invention.
{Fig. 12} Fig. 12 is a view showing an example of a reflecting member shown in Fig. 11.
{Fig. 13} Fig. 13 is a view showing, in outline, the configuration of an observation device according to a third embodiment of the present invention.
{Fig. 14} Fig. 14 is a view showing, in outline, the configuration of an observation device according to a modification of the third embodiment of the present invention.

### {Description of Embodiments}

### First Embodiment

An observation device according to this embodiment will be described below with reference to the drawings.

As shown in Fig. 1, an observation device 1 of this embodiment is provided with: a housing 5 on a top surface 5a of which a vessel 3 accommodating samples X can be placed; a light source unit 7, an image acquisition unit 9, and a focus adjustment unit 11 that are accommodated in the housing 5; a control unit 13 that controls the image acquisition unit 9 and the focus adjustment unit 11; a display unit 15 that displays an image acquired by the image acquisition unit 9; and a manipulation unit 17 that enables a user to input an instruction to be sent to the control unit 13.

The housing 5 is, for example, formed in a rectangular parallelepiped shape having a minimum roughness on outer surfaces thereof. With such an external form, the outer surfaces of the housing 5 can be easily subjected to wipe sterilization using 70% ethanol or the like. A glass window (transmissive window) 5b through which illumination light can be transmitted is provided in the top surface 5a of the housing 5 so as to cover an upper side of the light source unit 7 and the image acquisition unit 9.

Furthermore, except the glass window 5b, the housing 5 is made of a UV-resistant material, such as metal. With this material, the housing 5 can be sterilized by means of a UV germicidal lamp of a clean bench. For example, the housing 5 may be formed by using a hydrogen-peroxide-gas-resistant material, such as stainless steel or alumite. In this case, a gap on the boundary of the glass window 5b in the top surface 5a of the housing 5 or a gap of a hole through which a wire is made to pass may be sealed by a hydrogen-peroxide-gas-resistant material, such as silicon. By doing so, it is possible to deal with hydrogen-peroxide-gas sterilization.

Furthermore, the housing 5 is disposed such that the top surface 5a becomes substantially horizontal. The top surface 5a is disposed so as to be substantially horizontal, thereby making it possible to use the top surface 5a of the housing 5 as a workbench during non-observation. The glass window 5b, which is provided in the top surface 5a, has a larger area than the vessel 3, so that the vessel 3 can be placed on the glass window 5b.

The vessel 3 is, for example, a cell culture flask having a top plate 3a and is entirely made of an optically transparent resin.

The light source unit 7 is provided with a single LED light source 19 that is disposed, at a position shifted in a direction intersecting the optical axis of the image acquisition unit 9, so as to be opposed to the glass window 5b. The LED light source 19 emits illumination light obliquely upward, causes the illumination light to be transmitted through the glass window 5b and a bottom surface 3b of the vessel 3 upward from below, and causes the illumination light to be reflected at the top plate 3a of the vessel 3, thus radiating the illumination light onto the samples X in the vessel 3 from obliquely above. The LED light source 19, for example, emits illumination light of a red wavelength or higher. By doing so, phototoxicity for the samples X can be reduced.

The image acquisition unit 9 is provided with: an objective lens 21 that is disposed below the glass window 5b so as to be opposed to the glass window 5b; and a camera (camera unit) 23 that captures light focused by the objective lens 21. Illumination light from the LED light source 19 is radiated onto the samples X from above, thus being transmitted through the samples X downward from above and being transmitted through the glass window 5b downward from above, and the transmitted light entering the housing 5 is focused by the objective lens 21.

The focus adjustment unit 11 adjusts the focus state of the objective lens 21 with respect to the samples X in the vessel 3 through the control of the control unit 13.

The control unit 13 controls the focus adjustment unit 11 so as to match the focal point of the objective lens 21 with the samples X in the vessel 3, and automatically controls the exposure time and gain of the camera 23. When the user specifies the type of the vessel 3 by means of the manipulation unit 17, the control unit 13 controls the focus adjustment unit 11 to match the focal point of the objective lens 21 with the samples X in the vessel 3 in accordance with the type of the vessel 3.

Furthermore, the control unit 13 is capable of causing the display unit 15 to display an image acquired by the camera 23 and storing still images and moving images. The control unit 13 stores images, thus making it possible to keep acquired still images and moving images.

Furthermore, the control unit 13 is capable of processing an image to remove unevenness in brightness and to emphasize the contrast. Accordingly, it is possible to improve the visibility of the samples X. Furthermore, the control unit 13 can analyze an image to count the number of samples X. Accordingly, quantitative analysis of the samples X can be performed.

The display unit 15 is, for example, a liquid crystal display monitor. By using a liquid crystal display monitor, an image of the samples X can be displayed with good image quality.

The manipulation unit 17 is, for example, a mouse having buttons, a dial, a wheel, etc., and can be manipulated by the user with his or her hand. Accordingly, accurate manipulation is possible.

The operation of the thus-configured observation device 1 will now be described.

In order to observe the samples X, which are transparent like cells, by using the observation device 1 of this embodiment, as shown in Fig. 1, the samples X are accommodated in the vessel 3, the top plate 3a is closed, the vessel 3 is left to stand with the bottom surface 3b being located at a lower side, and, after the samples X adhere to the bottom surface 3b, the vessel 3 is placed on the glass window 5b in the top surface 5a of the housing 5.

Next, the LED light source 19 is actuated to generate illumination light. The illumination light generated by the LED light source 19 is transmitted, from a radially outer side of the objective lens 21, through the glass window 5b in the top surface 5a of the housing 5 and the bottom surface 3b of the vessel 3 upward from below, is reflected at an inner surface of the top plate 3a of the vessel 3, and is radiated onto the samples X from obliquely above.

Of the illumination light radiated onto the samples X, transmitted light that is transmitted through the samples X is transmitted through the bottom surface 3b of the vessel 3 and the glass window 5b of the housing 5 downward from above and enters the objective lens 21 in the housing 5. At this time, the illumination light is refracted and scattered due to the shapes of the samples X and the refractive index thereof or is dimmed due to the transmittance of the samples X, thus turning into transmitted light having information about the samples X, and the transmitted light is focused by the objective lens 21 and is captured by the camera 23. An image of the samples X acquired by the camera 23 is sent, by the control unit 13, to the display unit 15 and is displayed thereon.

In this way, according to the observation device 1 of this embodiment, the light source unit 7 and the image acquisition unit 9 are accommodated in the housing 5, on which the vessel 3 is placed, thereby making it possible to suppress interference of the light source unit 7 and the image acquisition unit 9 with work even when the observation device 1 is used in a limited space as in a clean bench. Furthermore, because the LED light source 19 and the image acquisition unit 9 are disposed below the samples X, it is possible to reduce the thickness of the housing 5 due to this arrangement in which the LED light source 19 and the image acquisition unit 9 are put together at only one side of the samples X, compared with a conventional transmitted-light observation device in which a light source unit and an image acquisition unit are disposed at both sides of the samples with the samples sandwiched therebetween. Accordingly, it is possible to improve the work efficiency in a limited space.

In this embodiment, the light source unit 7 may be provided with a diffusion plate (not shown) that diffuses illumination light generated by the LED light source 19. The diffusion plate is, for example, disposed between the LED light source 19 and the glass window 5b of the housing 5. By doing so, because the illumination light generated by the LED light source 19 is evenly diffused by the diffusion plate, the illumination light having uniform intensity and less unevenness in illumination can be radiated onto the samples X.

This embodiment can be modified as follows.

In this embodiment, although the light source unit 7 is provided with the single LED light source 19, in a first modification, for example, as shown in Fig. 2, the light source unit 7 may be provided with a plurality of LED light sources 19 disposed, around the objective lens 21, at intervals in the circumferential direction of the objective lens 21.

By turning on only the LED light source 19 that is disposed at a particular position in the circumferential direction of the objective lens 21, the samples X can be illuminated only from a particular direction in the circumferential direction. Furthermore, by simultaneously turning on the LED light sources 19 that are disposed in two or more directions in the circumferential direction of the objective lens 21, in particular, disposed in axially symmetric directions with respect to the optical axis of the objective lens 21, illumination light having reduced unevenness in illumination can be radiated onto the samples. In Fig. 2, although the two LED light sources 19 are shown as an example, the number of LED light sources 19 may be three or more.

In this modification, the plurality of LED light sources 19 may also be disposed at intervals not only in the circumferential direction of the objective lens 21 but also in a radial direction of the objective lens 21. In this case, a particular one of the LED light sources 19 may be turned on through control of the control unit 13. For example, by turning on only one of the LED light sources 19 disposed at different positions in the radial direction of the objective lens 21, the angle of illumination light to be radiated onto the samples X from obliquely upward can be changed.

In the first modification, although illumination light is emitted by switching between the plurality of LED light sources 19, instead of this, it is also possible to adopt light shielding members 25, shown in Fig. 3, that are disposed above the LED light sources 19 and that shield illumination light from the LED light sources 19.

As shown in Fig. 3, the light shielding members 25 are each provided with: an opening 25a that opens at a section in the circumferential direction or a section in the radial direction; and a transmission hole 25b for allowing transmitted light that has been reflected at the inner surface of the top plate 3a of the vessel 3 and that has been transmitted through the samples X, to pass therethrough. By changing between the plurality of light shielding members 25 having the openings 25a at different positions, it is possible to change the irradiation angle or the irradiation direction of illumination light according to the position of the opening 25a of each of the light shielding members 25.

In this case, although it is also possible to adopt, as in the first modification, the plurality of LED light sources 19 as the light source unit 7, it is not necessary to have a function of changing the position where illumination light is emitted, and it is possible to adopt a light source unit that is provided with an arbitrary light source as long as the light source can emit illumination light from a wider area than the opening 25a.

Furthermore, although the light shielding member 25, which has the circular opening 25a, is shown as an example in Fig. 3, instead of this, it is also possible to adopt an arbitrary size, position, and shape of the opening 25a, and an arbitrary number of openings 25a, for example, a fan-shaped opening 25a, an annular opening 25a, or the like.

Furthermore, in a second modification, for example, as shown in Fig. 4, an optical filter 27 that allows only the wavelength of illumination light to pass therethrough, such as a band-pass filter, may be disposed between the glass window 5b of the housing 5 and a distal end of the objective lens 21. By doing so, entry of external light, such as light from a fluorescent lamp in the clean bench, into the objective lens 21 can be prevented by the optical filter 27, thus making it possible to reduce deterioration of image quality caused by the influence of the external light.

Since the optical filter 27 merely needs to be able to remove external light that is about to enter the camera 23, the optical filter 27 may be provided, for example, inside the objective lens 21, instead of outside the objective lens 21, or may be provided between the objective lens 21 and the camera 23.

Furthermore, in this embodiment, although the display unit 15, such as a liquid crystal display monitor, has been described as an example display unit, in a third modification, it is also possible to adopt, as the display unit, a projector that projects an image. By doing so, the space for disposing the display unit separately from the housing 5 can be saved. Furthermore, the projector projects an image at a position, such as a wall opposed to the user with the observation device 1 therebetween, that the user can easily see in a posture adopted while working, thus making it possible to improve work efficiency.

Furthermore, it is also possible to adopt, as the display unit, a HUD (head-up display) that presents an image in the field of view of the user. By doing so, even when the user faces in an arbitrary direction, the user can work while always checking the image.

Furthermore, although the mouse has been shown as an example of the manipulation unit 17, instead of this, for example, as shown in Fig. 5, it is also possible to adopt a manipulation unit 29 that is manipulated by the user with his or her foot, such as a foot switch. By doing so, the user can input an instruction to the manipulation unit 29 while doing other work with his or her hands, thus making it possible to improve work efficiency. In each configuration, it is possible to adopt the manipulation unit 29, such as a foot switch, instead of the manipulation unit 17, such as a mouse, or to adopt the manipulation unit 17 instead of the manipulation unit 29. As the manipulation unit 17, a push-button input interface, such as a keyboard or a push-button remote controller, may also be adopted.

Furthermore, in a fourth modification, instead of the control unit 13 and the display unit 15, for example, as shown in Fig. 6, it is also possible to adopt a notebook PC (personal computer) 31 in which a control unit and a display unit are integrated. By doing so, image data acquired by the camera 23 can be directly used for advanced analysis and work by means of the notebook PC 31.

Furthermore, in a fifth modification, instead of the control unit 13, for example, as shown in Fig. 7, it is also possible to adopt a control unit 33 that is formed in an electronic board and that is mounted in the housing 5. By doing so, it is possible to simplify the configuration of the observation device 1 and to achieve power saving.

In a sixth modification, instead of the display unit 15 and the manipulation unit 17, for example, as shown in Fig. 8, it is also possible to adopt a display unit 35 and manipulation units 37 that are disposed in one surface of the housing 5. In this case, the housing 5 may have, on the top surface 5a, in addition to the glass window 5b, a space for disposing the display unit 35 and the manipulation units 37, and the display unit 35 and the manipulation units 37 may be disposed in the top surface 5a of the housing 5. Furthermore, it is also possible to adopt, as the control unit, the control unit 33 of the fifth modification. By doing so, it is possible to realize the simplest configuration in which the control unit 33, the display unit 35, and the manipulation units 37 are integrated with the housing 5.

In this modification, although the configuration in which the control unit 33, the display unit 35, and the manipulation units 37 are all integrated with the housing 5 is shown as an example, for example, it is also possible to use a configuration in which an integration of the housing 5, the control unit 33, and the manipulation units 37 and the display unit 15, which is a separate body, are adopted, or to use a configuration in which an integration of the housing 5, the control unit 33, and the display unit 35 and the manipulation unit 17, 29, which is a separate body, are adopted.

In a seventh modification, as shown in Fig. 9, the control unit 33, which is mounted in the housing 5, may perform transmission and reception with the display unit 15 and the manipulation unit 29 by wireless communication. In this case, the camera 23, the focus adjustment unit 11, and the control unit 33 may be battery-driven. Furthermore, still image data and moving image data acquired by the camera 23 may be sent to another PC 39 or a network (Network) 41 by the control unit 33 by wireless communication and may be stored in the other PC 39 or the network 41. By doing so, wiring is eliminated, thus making it possible to improve convenience.

In an eighth modification, as shown in Fig. 10, separately from the control unit 33, which is mounted in the housing 5 and which controls the image acquisition unit 9 and the focus adjustment unit 11, another control unit (image aggregation unit) 34 disposed outside the housing 5 may be provided.

The other control unit 34 can be connected to one or more other observation devices 2, the network (external recording medium) 41, the arbitrary other PC 39, etc., by wire or wirelessly. Furthermore, the other control unit 34 may receive image data of still images and moving images acquired in the observation device 1 of this embodiment or the other observation devices 2, from the observation device 1 or the other observation devices 2, may send the image data to the network 41, and may store the image data in the network 41.

According to this modification, the other control unit 34 functions as a hub (concentrator), puts image data from the observation device 1 together with image data from the other observation devices 2, and uploads the image data to a storage location on the network 41, thereby making it possible for the user to send an instruction or request to the network 41 from the arbitrary other PC 39 and to browse the image data. Accordingly, the samples X to be observed by means of the observation device 1 can be observed together with samples in the other observation devices 2, while being compared with them.

In this modification, it is also possible to send an instruction from the user to the control unit 33 and the other control unit 34 by using a manipulation unit 37 that is disposed on one surface of the housing 5, without adopting the manipulation unit 17, 29, or it is also possible to send an instruction from the user to the control unit 33 and the other control unit 34 by using both the manipulation unit 37 and the manipulation unit 17, 29, which is disposed outside the housing 5. Fig. 10 shows an example case in which the manipulation unit 37 and the manipulation unit 29 are both used. When the manipulation unit 37 and the manipulation unit 17, 29 are both used, the user can select an appropriate manipulation method for the work.

### Second Embodiment

Next, an observation device according to a second embodiment of the present invention will be described below with reference to the drawings.

As shown in Fig. 11, an observation device 51 of this embodiment differs from the observation device 1 of the first embodiment in that a reflecting member 53 that is disposed above the vessel 3 is provided.

In the description of this embodiment, identical reference signs are assigned to portions whose configurations are common to those in the observation device 1 of the above-described first embodiment, and a description thereof will be omitted.

The reflecting member 53 is, for example, disposed so as to be opposed to the glass window 5b and so as to cover the vessel 3 on the glass window 5b and reflects illumination light transmitted through the glass window 5b from the light source unit 7 upward from below, toward the samples X in the vessel 3. For example, a glass, a resin, a mirror, or the like is used as the reflecting member 53.

As shown in Fig. 12, the reflecting member 53 is configured so as to be capable of being withdrawn from above the vessel 3. The reflecting member 53 is disposed above the vessel 3 during observation, and the reflecting member 53 is withdrawn from above the vessel 3 after observation, thereby making it possible to prevent the reflecting member 53 from interfering with work when the top surface 5a of the housing 5 is used as a workbench.

A vessel that has a removable top plate 3c, like a petri dish, may be adopted as the vessel 3, thus allowing observation in a state in which the top plate 3c is removed.

The operation of the thus-configured observation device 51 will be described.

In order to observe the samples X, which are transparent like cells, by using the observation device 51 of this embodiment, first, the vessel 3, which accommodates the samples X, is placed on the glass window 5b of the housing 5, and then, the reflecting member 53 is disposed above the vessel 3. Then, illumination light is generated by the LED light source 19.

The illumination light generated by the LED light source 19 is transmitted through the glass window 5b in the top surface 5a of the housing 5 and the bottom surface 3b and the top plate 3c of the vessel 3 upward from below, is then reflected at the reflecting member 53, is transmitted through the top plate 3c of the vessel 3, and is radiated onto the samples X from obliquely above. The transmitted light transmitted through the samples X is transmitted through the bottom surface 3b of the vessel 3 and the glass window 5b of the housing 5 downward from above, enters the housing 5, is focused by the objective lens 21, and is captured by the camera 23.

According to the observation device 51 of this embodiment, because the illumination light from the LED light source 19 is reflected not at the top plate 3c of the vessel 3 but at the reflecting member 53 and is radiated onto the samples X, the illumination condition does not change with the type of the vessel 3, thus making it possible to stabilize the image quality.

In this embodiment, although the reflecting member 53 is adopted, it is also possible to pour a solution (for example, a culture medium, a phosphate buffer solution, or the like) into the vessel 3 to immerse the samples X in the solution and to reflect illumination light transmitted through the bottom surface 3b upward from below, at an upper liquid surface of the solution.

In this embodiment, as in the first embodiment and the modifications thereof, the control unit 13, 33, the display unit 15, 35, the manipulation unit 17, 29, 37, the notebook PC 31, and the other control unit 34 may be appropriately combined and adopted.

### Third Embodiment

Next, an observation device according to a third embodiment of the present invention will be described below with reference to the drawings.

An observation device 61 of this embodiment differs from the observation device 1 of the first embodiment and the observation device 51 of the second embodiment in that the housing 5 is embedded in a work floor 63a of a clean bench 63, thus being integrally formed with the work floor 63a, as shown in Fig. 13.

In the description of this embodiment, identical reference signs are assigned to portions whose configurations are common to those in the observation device 1 of the above-described first embodiment and the observation device 51 of the above-described second embodiment, and a description thereof will be omitted.

The housing 5 may be disposed such that the top surface 5a is flush with the work floor 63a. By doing so, during non-observation, the housing 5 does not interfere with work, and the top surface 5a of the housing 5 can be used as part of the work floor 63a.

Because an observation method for the samples X by using the thus-configured observation device 61 is the same as that of the first embodiment, except that manipulation is performed inside the clean bench 63, a description thereof will be omitted.

According to the observation device 61 of this embodiment, an observation function can be added to the clean bench 63, without sacrificing the workspace in the clean bench 63.

In this embodiment, as in the second embodiment, it is also possible to adopt the reflecting member 53 and to radiate illumination light from the light source unit 7 onto the samples X from above by means of the reflecting member 53. In this case, when the reflecting member 53 is made to be able to be withdrawn from above the samples X, the workspace in the clean bench 63 need not be sacrificed.

In this embodiment, instead of illumination light from the LED light source 19, as shown in Fig. 14, it is also possible to use illumination light from a bench illumination (light source) 65 of the clean bench 63 to observe the samples X. In this case, it is preferred that the control unit 13 remove flickering (flicker), caused by a fluorescent lamp, in an image sent from the camera 23, through image processing. By doing so, the illumination condition does not change with the capture environment, thus making it possible to stabilize the image quality.

In this case, it is also possible to dispose the optical filter 27, which allows only the wavelength of illumination light to pass therethrough, at one of a position between the glass window 5b and the objective lens 21, a position inside the objective lens 21, and a position between the objective lens 21 and the camera 23.

In this embodiment, although an example configuration in which the housing 5 is embedded in the work floor 63a has been described, instead of this, it is also possible to use a configuration in which the housing 5 is placed on the work floor 63a. In this case, because the light source unit 7 and the image acquisition unit 9 are accommodated in the housing 5, on which the vessel 3 is placed, interference of the light source unit 7 and the image acquisition unit 9 with work is suppressed even in the limited workspace in the clean bench 63, thus making it possible to improve work efficiency.

In this embodiment, as in the first embodiment and the modifications thereof, the control unit 13, 33, the display unit 15, 35, the manipulation unit 17, 29, 37, the notebook PC 31, and the other control unit 34 may be appropriately combined and adopted.

In the above-described respective embodiments, PCs can be adopted as the control unit 13 and the other control unit 34. A control circuit can be adopted as the control unit 33.

Although the embodiments of the present invention have been described in detail above with reference to the drawings, the specific configurations are not limited to the embodiments, and design changes etc. that do not depart from the scope of the present invention are also encompassed. For example, the present invention is not limited to the above-described respective embodiments and modifications, may be applied to an embodiment that is obtained by appropriately combining the embodiments and modifications, and is not particularly limited.

### {Reference Signs List}

- 1, 51, 61: observation device
- 2: another observation device
- 3: vessel
- 3a: top plate
- 5: housing
- 5b: glass window (transmissive window)
- 7: light source unit
- 9: image acquisition unit
- 11: focus adjustment unit
- 13, 33: control unit
- 15, 35: display unit
- 17, 29, 37: manipulation unit
- 19: LED light source (light source)
- 23: camera (camera unit)
- 21: objective lens
- 27: optical filter
- 31: notebook PC (control unit, display unit)
- 34: another control unit (image aggregation unit)
- 41: network (external recording medium)
- X: sample

## Claims

1. An observation device comprising:
a housing having, in a top surface, a transmissive window on which a vessel accommodating samples can be placed and through which illumination light can be transmitted; and
an image acquisition unit that is accommodated in the housing and that captures transmitted light obtained after the illumination light is transmitted through the samples from above, is transmitted through the transmissive window, and enters the housing.

2. The observation device according to claim 1, further comprising a light source unit that is accommodated in the housing and that emits the illumination light upward from below the samples,
wherein the image acquisition unit captures the transmitted light transmitted through the samples and the transmissive window after the illumination light emitted by the light source unit is reflected above the samples.

3. The observation device according to claim 2,
wherein the image acquisition unit includes an objective lens that focuses the transmitted light, and
the light source unit emits the illumination light from a radially outer side of the objective lens toward an upper side of the samples.

4. The observation device according to claim 3, wherein the light source unit can independently emit the illumination light from a different position in a radial direction of the objective lens.

5. The observation device according to claim 3, wherein the light source unit can simultaneously emit the illumination light from different positions in a circumferential direction of the objective lens.

6. The observation device according to claim 4 or 5, wherein the light source unit includes a plurality of light sources that are arranged around the objective lens and that can be independently turned on.

7. The observation device according to claim 3, wherein the light source unit includes:
a light source that is disposed below the samples; and
a light shielding member having an opening that allows, of the illumination light from the light source, only the illumination light at a particular radial position to pass therethrough.

8. The observation device according to one of claims 3 to 7,
wherein the image acquisition unit includes:
a camera unit that receives the illumination light focused by the objective lens and that acquires an image of the samples; and
an optical filter that allows only a wavelength of the illumination light to pass therethrough,
the objective lens is disposed so as to be opposed to the samples with the transmissive window sandwiched therebetween, and
the optical filter is disposed at one of a position between the transmissive window and the objective lens, a position inside the objective lens, and a position between the objective lens and the camera unit.

9. The observation device according to one of claims 2 to 8, wherein the light source unit includes a diffusion plate that diffuses the illumination light.

10. The observation device according to one of claims 2 to 9, wherein the illumination light is reflected by an inner surface of a top plate of the vessel.

11. The observation device according to one of claims 2 to 9, wherein the illumination light is reflected by a reflecting member disposed above the samples.

12. The observation device according to one of claims 2 to 9,
wherein the samples are immersed in a solution, and
the illumination light is reflected by an upper liquid surface of the solution.

13. The observation device according to claim 1, wherein the image acquisition unit captures the transmitted light transmitted through the samples and the transmissive window after the illumination light emitted by a light source disposed outside the housing is radiated onto the samples from above.

14. The observation device according to claim 13,
wherein the image acquisition unit includes:
an objective lens that focuses the transmitted light;
a camera unit that receives the illumination light focused by the objective lens and that acquires an image of the samples; and
an optical filter that allows only a wavelength of the illumination light to pass therethrough,
the objective lens is disposed so as to be opposed to the samples with the transmissive window sandwiched therebetween, and
the optical filter is disposed at one of a position between the transmissive window and the objective lens, a position inside the objective lens, and a position between the objective lens and the camera unit.

15. The observation device according to one of claims 1 to 14, further comprising a display unit that displays an image of the samples acquired by the image acquisition unit.

16. The observation device according to claim 15, wherein the display unit is disposed in one surface of the housing.

17. The observation device according to claim 15, wherein the display unit is a projector that projects the image.

18. The observation device according to one of claims 1 to 17, further comprising:
a focus adjustment unit that adjusts a focus state of the image acquisition unit with respect to the samples; and
a control unit that controls the focus adjustment unit so as to match a focal point of the image acquisition unit with the samples.

19. The observation device according to claim 18, wherein at least part of the control unit is contained in the housing.

20. The observation device according to claim 18 or 19, further comprising a manipulation unit that enables a user to input an instruction to be sent to the control unit.

21. The observation device according to claim 20, wherein the manipulation unit is a foot switch.

22. The observation device according to claim 20, wherein the manipulation unit is disposed on one surface of the housing.

23. The observation device according to one of claims 1 to 22, wherein the top surface of the housing is disposed so as to be substantially horizontal.

24. The observation device according to one of claims 1 to 23, further comprising an image aggregation unit that puts an image of the samples acquired by the image acquisition unit together with an image acquired by another observation device and that stores the images in an external recording medium.
